# EUROPEAN PATENT APPLICATION

(11) **EP 4 344 664 A1**
(43) Date of publication of application: **03.04.2024**
(21) Application number: 22198299.4
(22) Date of filing: 28.09.2022
(51) Int. Cl.: A61B 18/18

(54) **PHOTO-EPILATOR DEVICE**

(71) Applicant: Koninklijke Philips N.V., 5656 AG Eindhoven (NL)
(72) Inventor: VERKRUIJSSE, Willem, Eindhoven (NL); THUMMA, Kiran Kumar, Eindhoven (NL); VARGHESE, Babu, 5656AG Eindhoven (NL); BOURQUIN, Yannyk Parulian Julian, 5656AG Eindhoven (NL); PALERO, Jonathan Alambra, 5656AG Eindhoven (NL)
(74) Representative: Philips Intellectual Property & Standards

(57) **Abstract**

A photo-epilator uses an intense pulsed light, IPL, lamp to deliver pulses of energy. In a skin preparation mode, desensitizing pulsed light energy is used and in an epilation mode, therapeutic pulsed light energy is used. The pulse or pulses of the skin preparation mode use pulsed energy characteristics which result in a lower pulse energy than in the epilation mode.

## Description

### FIELD OF THE INVENTION

This invention relates to hair treatment devices for hair removal or hair growth reduction by photo-epilation.

### BACKGROUND OF THE INVENTION

Light therapy is known for hair removal and hair growth reduction.

Home-use photo-epilation consumer devices are commercially available, such as the Lumea (TM) of Philips (TM). Home-use devices typically take the form of a hand held device using Intense Pulsed Light technology (IPL) from e.g. a Xenon flash lamp at a relatively low fluence (e.g., 6.5 J/cm²), as compared to professional devices for permanent photo-epilation, that use fluences in excess of 10 J/cm².

Light is absorbed by the hair roots and the hair follicles present in the skin, which are considerably heated as a result of the relatively high energy density of the light. By using a proper configuration of the light energy, namely the wavelength, intensity, and pulse duration, selective heating of the hair root and the desired temporary or permanent damage to the hair follicle can be achieved.

The IPL technology uses the flash lamp to deliver an intense, visible, broad-spectrum pulse of light, generally in the visible spectral range of 400 to 1200 nm. Cutoff filters are for example used to selectively filter out shorter wavelengths, especially potentially damaging ultra violet light. The resulting light has a spectral range that targets specific structures and chromophores, in particular the melanin pigment in hair. IPL shares some similarities with laser treatments, in that they both use light to heat and destroy their targets. Unlike lasers that use a single wavelength of light which typically matches only one chromophore and hence only treats one condition, IPL uses a broad spectrum.

The light absorbed by melanin in the hair and hair matrix generates heat that damages the hair follicles. When the treatment is repeated in intervals of 2 to 4 weeks, a long-lasting hair reduction result is obtained.

However, due to the high fluence required to achieve sufficient temperature elevation in the follicle, the skin is warmed, and this can cause discomfort to the user. This negatively affects the user perception. To reduce the level of pain, the light energy per pulse to which the skin is exposed may be reduced, but this would also reduce the therapeutic efficacy. Alternatively, a specific pre-treatment of the targeted are may be performed to reduce the pain.

Such pre-treatment methods include vibration or using a dynamic cooling device such as a cryogen spray system. Alternatively, the use of heat stimulus to desensitize receptors in the skin has been proposed in WO2019/173623A1. WO2019/173623 discloses a system which aims to treat pain by pulsating heat into a volume of tissue to induce desensitization of thermos-receptors, using a conductive, convective or a radiation heating element. However, the solution requires a treatment cycle with ramp up period, a steady period and a ramp down period. In IPL, the flash is instead instantaneous, and it is followed by a free decay.

There remains a need for a photo-epilator system which reduces discomfort while retaining the desired hair removal efficacy, and which can be applied to flash treatment system without adding significant complexity to the system.

### SUMMARY OF THE INVENTION

The invention is defined by the claims.

According to examples in accordance with an aspect of the invention, there is provided a photo-epilator device comprising:
an intense pulsed light, IPL, lamp; and
a controller for driving the IPL lamp to deliver pulses of energy,
wherein the controller is configured:
   in a skin preparation mode, to drive the IPL lamp to deliver desensitizing pulsed light energy with first pulsed energy characteristics; and
   in an epilation mode, following the skin preparation mode, to drive the IPL lamp to deliver therapeutic pulsed light energy with second pulsed energy characteristics, different to the first energy characteristics, wherein the first pulsed energy characteristics result in a lower pulse energy than the second pulsed energy characteristics.

This device uses the IPL lamp itself to provide a skin preparation function before the epilation, in order to reduce pain associated with the epilation. A lower pulse energy is used in the skin preparation mode. The "pulse energy" is in particular the pulse energy delivered to a given area of the skin, i.e., the fluence. This lower pulse energy avoids pain in the skin preparation mode itself, and also reduces pain in the subsequent higher energy epilation mode, by desensitizing the receptors in the skin. The pain reduction is thus achieved without significant change in hardware and hence cost and also does not significantly prolong the overall treatment.

The time period between the preparation mode and the epilation mode is for example in the range 10ms to 500ms.

The pulses in the epilation mode for example have a duration in the range 5ms to 100ms, for example 5ms to 20ms and the pulse or pulses of the skin preparation mode may have a duration of a similar order of magnitude, for example in the range 0.1ms to 10ms.

A single pulse may be used for the preparation mode, or there may be a set of pulses. These may be considered to be desensitizing pulses.

The first pulsed energy characteristics for example result in a fluence less than half the fluence resulting from the second pulsed energy characteristics.

For example, the fluence resulting from the first pulsed energy characteristics may be less than 15 J/cm² (e.g., 10 J/cm² or less, or less than 8 J/cm²) and the fluence resulting from the second pulsed energy characteristics may be more than 20 J/cm² (e.g., 30 J/cm²).

The first pulsed energy characteristics for example result in a fluence less than 0.45 times, or less than 0.4 times, or less than 0.35 times the fluence resulting from the second pulsed energy characteristics.

The first and second pulsed energy characteristics for example differ by one or more of:
a pulse peak wavelength;
an emission frequency spectrum;
a pulse peak intensity;
a pulse duration;
a pulse delay; and
a pulse temporal profile;

These are frequency or temporal characteristics of the desensitizing pulses of the preparation mode and therapeutic pulses of the epilation mode.

The first and second pulsed energy characteristics may differ by one or more of:
a pulse beam diameter;
a pulse beam divergence; and
a pulse beam spatial distribution.

These are spatial characteristics of the desensitizing pulses of the preparation mode and therapeutic pulses of the epilation mode, which will therefore give rise to different energy delivery at different skin locations.

The device may further comprise a feedback sensor for sensing at least one skin parameter, and the controller is configured to adapt the first and/or second pulsed energy characteristics in response to the skin parameter.

This is performed to optimize the desensitization and/or the epilation efficacy. For example, different desensitization or therapy settings may be appropriate for different skin areas.

The at least one skin parameter for example comprises a melanin index. Thus, the desensitization or therapy may be dependent on the local skin color/pigmentation.

The device may comprise a pain detection sensor for detecting a pain level and the controller is configured to adapt the first pulsed energy characteristics in response to the pain level.

Thus, the desensitizing pulses of the preparation mode may be adapted based on the pain experienced during the preparation mode itself (to prevent the preparation mode being painful) and/or during the epilation (to make sure correct preparation is being performed to manage pain during the therapy).

The controller may be configured to implement a calibration phase during which the first pulsed energy characteristics are adapted.

This is used to personalize and optimize the efficacy of the desensitization during the preparation mode. The calibration results can be stored for the next use (of that particular user) such that a calibration is not needed each time the device is used.

The calibration phase for example comprises:
progressively increasing pulse energy for energy pulses to be applied;
receiving a user indication relating to the sensing of the pulse; and
setting a pulse energy in response to the user indication.

The pulse energy can be progressively increased by increasing the pulse duration or the peak power. The user is for example instructed (via audio, or otherwise) to stop when the pulse is felt, but not painful. This level is then used in the remainder of the therapeutic procedure. The calibration procedure may also be used to determine the time between the preparation pulse and the therapeutic pulse.

The calibration phase may comprise receiving a user indication for different skin areas and setting different pulse energies for different skin areas.

A body localization unit may be used to store the best first pulsed energy characteristics (desensitizing pulse characteristics of the preparation mode) per body location.

The device is for example a hand-held photo-epilator.

The invention also provides a computer program comprising computer program code which is adapted, when said program is run by a controller of a photo-epilator having an IPL lamp, to perform a method comprising:
in a skin preparation mode, driving the IPL lamp to deliver desensitizing pulsed light energy with first pulsed energy characteristics; and
in an epilation mode, following the skin preparation mode, driving the IPL lamp to deliver therapeutic pulsed light energy with second pulsed energy characteristics, different to the first energy characteristics, wherein the first pulsed energy characteristics result in a lower pulse energy than the second pulsed energy characteristics.

The first pulsed energy characteristics for example result in a fluence less than half the fluence resulting from the second pulsed energy characteristics.

The first and second pulsed energy characteristics for example differ by one or more of:
a pulse peak wavelength;
an emission frequency spectrum;
a pulse peak intensity;
a pulse duration;
a pulse temporal profile;
a pulse beam diameter;
a pulse beam divergence; and
a pulse beam spatial distribution.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows known pulse generation circuitry and an IPL pulse (as current versus time);
Fig. 2 shows the addition of control electronics between the energy storage capacitor and the lamp and shows a desired block pulse; and
Fig. 3 shows an example of light output pulses in accordance with the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a photo-epilator which uses an intense pulsed light, IPL, lamp to deliver pulses of energy. In a skin preparation mode, desensitizing pulsed light energy is used and in an epilation mode, therapeutic pulsed light energy is used. The pulse or pulses of the skin preparation mode use pulsed energy characteristics which result in a lower pulse energy than in the epilation mode.

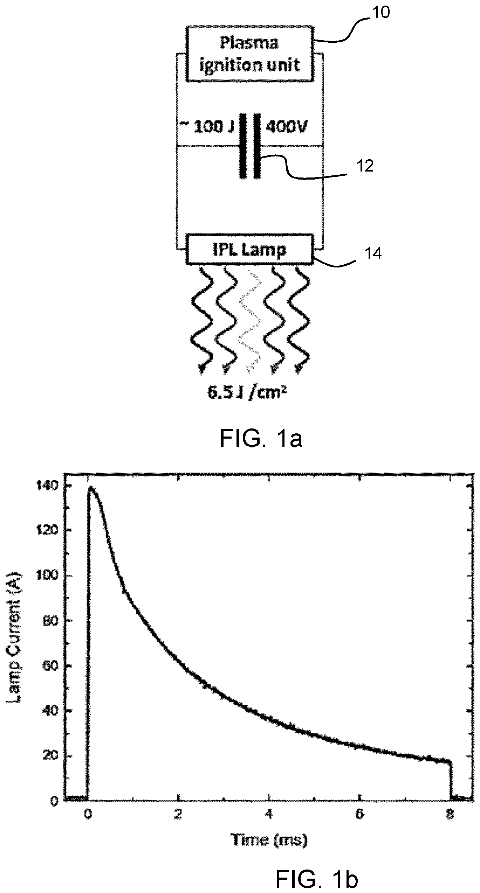

Fig. 1a shows an example of known pulse generation circuitry for a photo-epilator, and Fig. 1b shows the IPL pulse (as current versus time), as implemented in the Lumea (TM) system. The pulse for example delivers a fluence of approximately 30J/cm² of energy to the skin surface.

Plasma is ignited by a plasma ignition unit 10. Energy is stored in a main storage capacitor 12 (e.g. 100J of energy at 400V). The electrical energy stored in the capacitor is discharged through the lamp 14, resulting in a pulsed operation of the lamp. The lamp is for example a gas-filled linear flash lamp.

The plasma ignition unit 10 generates a conductive plasma channel of ionized gas atoms or molecules between the lamp electrodes (inside the lamp tube). At first, a high voltage (of around 15kV) is applied between the lamp electrodes. The high voltage is needed to generate the electrical breakdown of the gas inside the lamp. The high voltage cannot however deliver the electrical charge needed to open a conductive plasma channel.

For this, a boost capacitor may be used. This boost capacitor delivers the required electrical charge. It only stores a small amount of electrical energy, about 1-2% of the electrical energy compared to the main capacitor, while its electrical voltage is around 800V.

The timing sequence of the high voltage, boost capacitor discharge and the main capacitor discharge are very precise for the lamp discharge to take place. In particular, the three steps overlap; the boost capacitor discharge starts while the gas breakdown is still present and the main capacitor discharge is initiated before the boost capacitor discharge is finished.

The lamp is for example fitted within a head of the hair treatment device with a radiation exit opening. During operation, the lamp generates light pulses having a relatively high energy density, which propagate towards the radiation exit opening and irradiate the human skin present in front of the radiation exit opening. Part of the light is absorbed by the hair roots and the hair follicles present in the skin, which are considerably heated as a result of the relatively high energy density of the light. As a result the hair roots and the hair follicles are damaged or even destroyed, so that growing of the hairs is prevented for a considerably long time or even permanently.

As shown by the IPL pulse shape in Fig. 1b, the current rises very rapidly to its maximum and then decays exponentially as the energy in the capacitor is consumed and the light pulse is generated. This type of discharge is an exponential decay pulse, as the current (as well as the light intensity output of the IPL lamp) follows the form of an exponential decay during the IPL flash. In the example shown, the current is stopped after 8ms, by means of a power transistor.

The use of this type of pulse gives an easy implementation, as it is cost effective, and a minimal physical volume is occupied by the used components. However, the large light output intensity at the beginning of the pulse (caused by the initial lamp current peak) can be a contributor to the discomfort for the user. Some users describe the IPL flash as painful and some others as a sharp unpleasant sting.

It is known to make the light output during the IPL pulse more even, ideally with a so-called block pulse, in particular by adapting the lamp current so that it is more constant over the duration of the IPL pulse, instead of having a high initial peak shown in Fig. 1b.

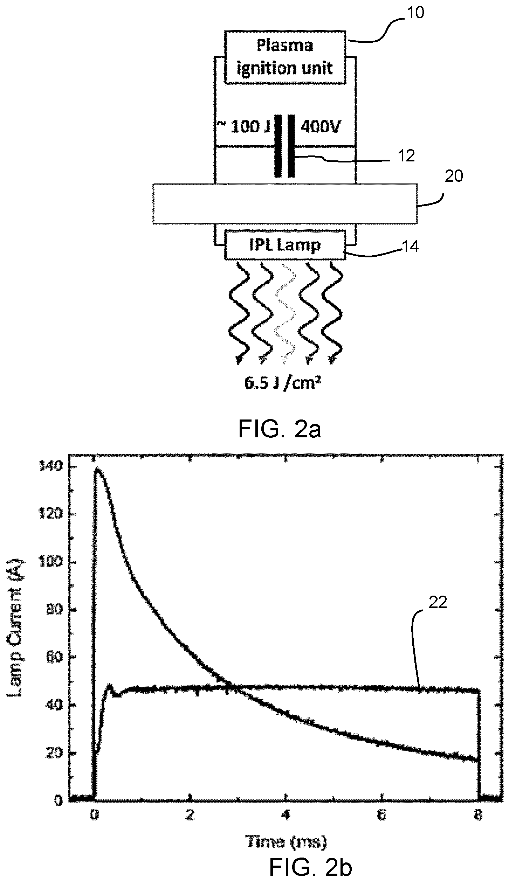

Fig. 2a shows the addition of a controller 20 between the energy storage capacitor 12 and the lamp 14, and shows a block pulse 22 which can be generated in Fig. 2b. The more uniform lamp current during the IPL pulse is beneficial for the user comfort. It also opens the way for treatment personalization and treatment efficiency benefits, as proven in professional IPL devices.

The block pulse can for example be implemented using an inductive coil and using a switch circuit to implement current modulation.

The invention may be applied when free delay pulses are being used (Fig. 1b) or when block pulses are being used (Fig. 2b).

The invention makes use of a controller, such as the controller 20 of Fig. 2a, so that the lamp can be controlled with different settings at different times, so that different modes of operation can be implemented.

In particular, the controller implements a skin preparation mode and an epilation mode.

In the preparation mode, the IPL lamp delivers desensitizing pulsed light energy with first pulsed energy characteristic and in the epilation mode, the IPL delivers therapeutic pulsed light energy with second pulsed energy characteristics, different to the first energy characteristics. The first pulsed energy characteristics result in a lower pulse energy than the second pulsed energy characteristics. The skin preparation mode may be considered to be a skin pre-heating mode or a skin desensitizing mode.

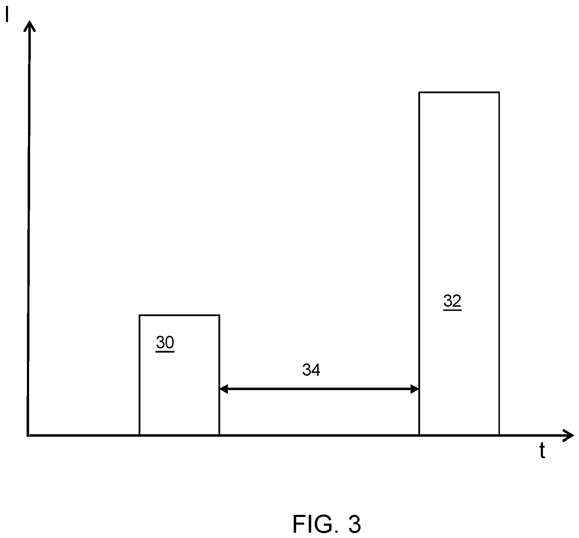

Fig. 3 shows a plot of lamp output overtime. For simplicity, a block waveform is shown.

The light output comprises a first pulse 30, which is a desensitizing pulse of the preparation mode. By way of example, the first pulse 30 delivers a pulse energy (per unit area of skin, i.e. a fluence) of 10 J/cm². The first pulse has first pulsed energy characteristics.

The preparation mode may comprise a single pulse to desensitize the skin, but there may instead by multiple pulses in the preparation mode.

A second pulse 32 is a therapeutic pulse of the epilation mode. By way of example, the second pulse 32 delivers a pulse energy of 30 J/cm². The second pulse has second pulsed energy characteristics.

The therapy typically involves the delivery of a single therapeutic pulse per skin location and per treatment session. However, there could instead be a sequence of the therapeutic pulses per location during each treatment session.

The number of light pulses (flashes) in a treatment session will depend on the surface area of skin to cover.

In a conventional IPL treatment, there is thus a single pulse per skin location within a treatment session. The single pulse is repeated for different skin locations. The invention instead provides (at least) two pulses for each skin location for a single treatment session - a preparation (desensitizing) pulse and a therapeutic pulse. The two pulses have different energy characteristics, i.e. different intensity vs. time pulse shapes. There could also be a sequence of the first (preparation) pulses per location during each treatment session and/or a sequence of the second (therapeutic) pulses per location during each treatment session. However, in a preferred implementation there is a single preparation pulse and a single therapeutic pulse per skin location per treatment session.

The time period 34 between the end of the (last) pulse in the preparation mode and the (first) pulse of the epilation mode is for example in the range 2ms to 500ms, preferable 10ms to 500ms, for example 10ms to 100ms.

Fig. 3 schematically shows the different energy levels being implemented simply as different light intensities. This is one way to deliver different amounts of energy. However, the pulses may differ in other ways.

For example, various spectral (frequency), amplitude, or temporal characteristics of the desensitizing pulses of the preparation mode may be different to the therapeutic pulses of the epilation mode to alter the energy delivery characteristics.

For example, the first and second pulsed energy characteristics for example differ by one or more of:
a pulse peak wavelength;
an emission frequency spectrum;
a pulse peak intensity;
a pulse duration; and
a pulse temporal profile.

As well as, or instead of, the energy characteristics at specific points in space, spatial characteristics may be adapted. For example, a fluence may be reduced by spreading a same pulse over a larger area. The first and second pulsed energy characteristics may for example differ by one or more of:
a pulse beam diameter;
a pulse beam divergence; and
a pulse beam spatial distribution.

In one example, the emission spectrum differs between the two modes. In the preparation mode, the cut-off filter is at 450 nm and in the epilation mode the cut-off filter is at 560 nm. In this case, the preparation mode would contain wavelengths absorbed by blood and will be able to stimulate the pain receptor with lower energy. Alternatively, two light sources (e.g. LEDs) may be used for the two different modes.

In another example, the beam diameter of the preparation mode may be smaller to reduce the amount of energy required to stimulate at least part of the pain receptor in a skin area and the epilation mode would have a larger beam to treat a large area of the skin.

Fig. 3 shows the pulses 30, 32 with the same duration. As will be clear from the discussion above, the fluence can be controlled using the pulse duration. The therapeutic pulse for example has a duration in the range 5ms to 100ms, for example 5ms to 20ms (8ms being a typical known therapeutic pulse duration for a low intensity hand held photo-epilator). The desensitizing pulse for example has a duration in the range 0.1ms to 10ms.

The device of the invention uses the IPL lamp itself to provide a skin preparation function before the epilation, in order to reduce pain associated with the epilation. A lower pulse energy (in particular the fluence, hence energy per unit of skin area) is used in the skin preparation mode.

The first pulsed energy characteristics for example result in a fluence less than half the fluence resulting from the second pulsed energy characteristics (at a given target skin location), for example less than 0.4 times, or less than 0.35 times or less than a third of the fluence resulting from the second pulsed energy characteristics.

The lower pulse energy avoids pain in the skin preparation mode itself, and also reduces pain in the subsequent higher energy epilation mode, by desensitizing the receptors in the skin. The pain reduction is thus achieved without significant change in hardware and hence cost and also does not significantly prolong the overall treatment.

The preparation mode may be personalized to the particular user, in particular because the pain sensation is user-specific and may also vary for different skin areas.

One approach is to provide feedback sensing of a skin parameter, such as a level of pigmentation detected based on the melanin index. This may be measured using an optical sensor such as a camera.

Thus, the pulsed energy characteristics during the preparation mode and/or during the epilation may be made dependent on the local skin color/pigmentation. This may enable the desensitization and/or the epilation efficacy to be improved, for example by having different desensitization or treatment settings for different skin areas.

A pain detection sensor may be used for detecting a pain level. A user may report a pain level using a user interface, but pain may also be measured using a sensor. For example, the bispectral index (BIS) may be measured using electrodes placed on the frontal and temporal areas. An algorithm interprets cortical activity (EEG) and corrugator supercilia muscle activity (EMG).

The pupillary dilatation reflex (PDR) may be measured.

Vital signs may also be used to detect pain levels, such as blood pressure, heart rate, respiratory rate. Skin conductance fluctuations (NSCF) can be measured using electrodes placed on palmar surface.

The interpretation of these different signals to derive a pain assessment will be known to those skilled in the art and does not form part of this invention.

The pain assessment may be used to adapt the first pulsed energy characteristics of the preparation mode. For example, if the preparation mode itself is causing pain (as well as the therapy), the preparation energy fluence may be reduced. If the pain is detected during therapy (but not during preparation), a higher energy fluence may be used during preparation.

Since the pain sensation is user-specific, a calibration phase may be used to determine the suitable energy characteristics for the pulses of the preparation mode, and optionally also for the epilation mode. This calibration can be used to personalize and optimize the efficacy of the desensitization during the preparation mode.

In one possible calibration procedure, the user is instructed (via audio or otherwise) to apply the hand held device to a sequence of different locations, and the device applies a stepwise increase in pulse energy. The energy level can be set by increasing the pulse duration or the peak power. The user is instructed to stop when the pulse is desensitization pulse felt, but is not painful. This level can then be used in the remainder of the therapeutic procedure.

The calibration procedure may also be used to determine the time between the preparation (desensitization) pulse and the therapeutic pulse.

A too short time would make the preparation pulse and therapeutic pulse be felt as a single pulse due to the thermal relaxation, while a too long time between pulses could lead to an effect called temporal summation where the pain is cumulative (i.e. two sequential stimulus that would not be painful alone would become painful when applies sequentially). This happens when the time between the two modes is at least 500ms. Thus, there is a window for the time between the two modes of approximately 2ms to 500ms that can be used. As mentioned above, the time delay is for example in the range 10ms to 100ms.

However, the perception of each individual may differ due to physiological aspects, so that a calibration may improve the results. In particular, differences in skin tone, hair density and body location may have an impact on the most optimal time between two modes. Thus, the time delay may be set as part of the calibration procedure and it may be different for different skin areas.

This calibration procedure may be repeated for different skin areas with different pain levels (either due to anatomical location or due to pigmentation variations.)

By storing the calibration results for each user, the calibration may be performed only once, or infrequently (i.e. less frequently than the treatment). By using position sensing, the device can determine its position relative to the user so that the device can sense the area of skin being treated, and hence map the skin-location-specific calibration settings to the location of the device. The use of skin color sensing as mentioned above also enables different energy characteristics to be used for areas of different skin pigmentation.

In a more basic approach, without requiring feedback sensing or calibration, the pulse (or pulses) of the preparation mode may apply a fluence which is a set fraction of the fluence of the therapeutic pulse.

Devices typically have a number of settings to allow the user to select their desired treatment level, e.g. settings 1 to 5. The preparation mode may for example simply use a lower energy setting than the epilation mode, For example, if the user chooses therapeutic pulses at setting 5 (of a 1 to 5 scale), the preparation mode may automatically apply pulses corresponding to setting 3 or 4. The setting for the preparation pulse relative to the chosen therapy pulse setting may be determined experimentally in a lab, on a range of volunteers.

The device is for example a hand-held photo-epilator but the invention may be applied to other epilator devices.

The invention may be implemented in software, as a computer program which is run by a processor of the controller 20, in order to control the way the lamp is driven.

Some of the energy characteristics may be controlled by suitable control of the lamp drive current, such as the a pulse peak intensity, the pulse duration, the pulse delay and the pulse temporal profile.

Other characteristics may controlled by additional control elements.

For example, the pulse peak wavelength and emission frequency spectrum may be adjusted by changing a cut-off filter, or controlling a temperature of a cut-off filter, or switching between different light sources for the different modes.

The pulse beam diameter, pulse beam divergence and a pulse beam spatial distribution may for example be adjusted by using a controllable beam expander, or actuated collimating optics (such as a liquid lens, diaphragm or tunable lens).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A photo-epilator device comprising:
an intense pulsed light, IPL, lamp (14); and
a controller (20) for driving the IPL lamp to deliver pulses of energy,
wherein the controller is configured:
in a skin preparation mode, to drive the IPL lamp to deliver desensitizing pulsed light energy with first pulsed energy characteristics; and
in an epilation mode, following the skin preparation mode, to drive the IPL lamp to deliver therapeutic pulsed light energy with second pulsed energy characteristics, different to the first energy characteristics, wherein the first pulsed energy characteristics result in a lower pulse energy than the second pulsed energy characteristics.

2. The device of claim 1, wherein the pulse or pulses in the epilation mode have a duration in the range 5ms to 100ms, for example 5ms to 20ms, and the pulse or pulses of the skin preparation mode have a duration in the range 0.1ms to 10ms.

3. The device of claim 1 or 2, wherein a delay between the skin preparation mode and the epilation mode is in the range 2ms to 500ms, for example 10ms to 500ms, for example 10ms to 100ms.

4. The device of any one of claims 1 to 3, wherein the first pulsed energy characteristics result in a fluence less than half the fluence resulting from the second pulsed energy characteristics.

5. The device of any one of claims 1 to 4, wherein the first and second pulsed energy characteristics differ by one or more of:
a pulse peak wavelength;
an emission frequency spectrum;
a pulse peak intensity;
a pulse duration; and
a pulse temporal profile.

6. The device of any one of claims 1 to 5, wherein the first and second pulsed energy characteristics differ by one or more of:
a pulse beam diameter;
a pulse beam divergence; and
a pulse beam spatial distribution.

7. The device of any one of claims 1 to 6, further comprising a feedback sensor for sensing at least one skin parameter, such as a melanin index, and wherein the controller is configured to adapt the first and/or second pulsed energy characteristics in response to the skin parameter.

8. The device of any one of claims 1 to 7, comprising a pain detection sensor for detecting a pain level and wherein the controller is configured to adapt the first pulsed energy characteristics in response to the pain level.

9. The device of any one of claims 1 to 8, wherein the controller is configured to implement a calibration phase during which the first pulsed energy characteristics are adapted.

10. The device of claim 9, wherein the calibration phase comprises:
progressively increasing pulse energy for energy pulses to be applied;
receiving a user indication relating to the sensing of the pulse; and
setting a pulse energy in response to the user indication.

11. The device of claim 10, wherein the calibration phase comprises:
receiving a user indication for different skin areas and setting different pulse energies for different skin areas.

12. The device of any one of claims 1 to 11, comprising a hand-held photo-epilator.

13. A computer program comprising computer program code which is adapted, when said program is run by a controller of a photo-epilator device having an IPL lamp, to perform a method comprising:
in a skin preparation mode, driving the IPL lamp to deliver desensitizing pulsed light energy with first pulsed energy characteristics; and
in an epilation mode, following the skin preparation mode, driving the IPL lamp to deliver therapeutic pulsed light energy with second pulsed energy characteristics, different to the first energy characteristics, wherein the first pulsed energy characteristics result in a lower pulse energy than the second pulsed energy characteristics.

14. The computer program of claim 13, wherein the first pulsed energy characteristics result in a fluence less than half the fluence resulting from the second pulsed energy characteristics.

15. The computer program of claim 13 or 14, wherein the first and second pulsed energy characteristics differ by one or more of:
a pulse peak wavelength;
an emission frequency spectrum;
a pulse peak intensity;
a pulse duration;
a pulse temporal profile;
a pulse beam diameter;
a pulse beam divergence; and
a pulse beam spatial distribution.
